# FASCICULE DE BREVET EUROPEEN

(11) **EP 2 273 979 B1**
(45) Date de publication et mention de la délivrance du brevet: **24.09.2014**
(21) Numéro de dépôt: 09742254.7
(22) Date de dépôt: 07.04.2009
(51) Int. Cl.: A61K 9/14, A23L 1/236

(54) **COMPOSITION PULVERULENTE DE MALTITOL CRISTALLISÉ DE GRANDE FLUIDITE ET NON MOTTANTE**
PULVERFÖRMIGE UND KRISTALLINE MALTITOLZUSAMMENSETZUNG VON HOHER FLIESSFÄHIGKEIT UND OHNE KLUMPENBILDUNG
HIGH-FLUIDITY AND NON-CAKING PULVERULENT CRYSTALLINE MALTITOL COMPOSITION

(30) Priorité: 08.04.2008 FR 0852352
(43) Date de publication de la demande: 19.01.2011
(73) Titulaire: Roquette Freres, 62136 Lestrem (FR)
(72) Inventeur: LEFEVRE, Philippe, F-59660 Haverskerque (FR); LIS, José, F-59253 La Gorgue (FR); RIBADEAU-DUMAS, Guillaume, F-59237 Verlinghem (FR)
(74) Mandataire: Cabinet Plasseraud
(86) Numéro de dépôt international: PCT/FR2009/050591
(87) Numéro de publication internationale: WO 2009/136056

(56) Documents cités:
- EP-A- 0 664 960
- EP-A1- 0 662 285
- EP-A2- 1 738 657
- US-A1- 2004 052 897
- US-A1- 2005 118 316

## Description

La présente invention est relative à une composition pulvérulente de maltitol cristallisé dont la fluidité est préservée. Cette poudre de maltitol cristallisé présente ainsi la particularité de ne pas être sujette au mottage. La composition selon l'invention présente avantageusement une fine granulométrie.

Au sens de l'invention, on entend par « composition pulvérulente de maltitol cristallisé », une poudre contenant du maltitol cristallisé.

Au sens de l'invention, on entend également par « maltitol cristallisé », le produit de la cristallisation classique d'une solution aqueuse de maltitol ou tout autre maltitol sous forme cristalline.

Le 4-O-alpha-D-glucopyranosyl-D-glucitol, appelé communément maltitol, est un polyol obtenu industriellement par hydrogénation du maltose.

Il présente un grand intérêt en raison du fait qu'il est plus stable chimiquement, moins calorique et qu'il présente un index glycémique plus bas que le saccharose, tout en possédant avantageusement des propriétés organoleptiques très voisines de celles de ce sucre.

De plus, le maltitol possède la particularité de n'être pas cariogène, ce qui lui ouvre et lui a déjà ouvert de multiples applications dans l'industrie, notamment dans les industries pharmaceutiques et alimentaires, notamment dans les domaines du chewing gum, des édulcorants de table et du chocolat.

Pendant très longtemps, le maltitol n'a été commercialisé que sous la forme de sirops de faible richesse, ou sous la forme de poudres amorphes et impures.

Ce n'est qu'au début des années 1980, que la société HAYASHIBARA décrivait pour la première fois dans son brevet US 4.408.041 la production de cristaux de maltitol anhydre.

Auparavant, ce polyol avait toujours été considéré comme un produit non cristallisable.

Ce postulat erroné trouve en réalité son origine dans le fait que la cristallisation du maltitol à partir d'une solution sursaturée n'est pas aussi facile à contrôler que dans le cas d'autres polyols comme le mannitol ou l'érythritol par exemple.

Les techniques dites de "massé" d'une part et de cristallisation dans l'eau d'autre part, sont aujourd'hui quasiment les seuls procédés employés industriellement.

Les produits ainsi obtenus ont cependant une cristallinité très variable, et ne conviennent pas tous particulièrement bien à certaines applications comme celles du chewing-gum ou du chocolat.

Ces produits cristallisés ne sont pas non plus totalement satisfaisants lorsque par exemple on souhaite utiliser du maltitol pour remplacer le saccharose ou le lactose dans les formes sèches pharmaceutiques telles que les gélules, les médicaments du type poudres à dissoudre, les comprimés et les préparations nutritives pulvérulentes à diluer.

C'est également le cas lorsque l'on souhaite réaliser le même genre de substitution dans les aliments sucrés tels que les boissons en poudre, les entremets, les préparations pour gâteaux ou les poudres chocolatées ou vanillées pour petit déjeuner.

On constate pour ces applications particulières, notamment pour les poudres pseudo-cristallines de maltitol obtenues par la technique de "massé", et à un degré moindre pour les poudres cristallines de maltitol obtenues par cristallisation dans l'eau, que celles-ci présentent un ou plusieurs défauts comme en particulier ceux de :
- s'écouler difficilement,
- d'être sujettes à une prise en masse ou à un mottage,
- de ne se dissoudre que très lentement dans l'eau.

Les spécialistes du domaine de la cristallisation des polyols se sont donc attachés à mettre au point des compositions de maltitol n'ayant pas les défauts notamment d'écoulement et de mottage que présentent les poudres de maltitol connues.

D'une manière générale, les polyols proposés sous la forme de produits pulvérulents sont entreposés et distribués dans des emballages doubles associant un sac interne fait d'une matière plastique à un sac en papier Kraft ou à une boîte en carton ondulé ou encore en conteneurs souples dits « big bags ». Le conditionnement du maltitol cristallin poudre actuellement commercialisé fait appel à l'un ou l'autre de ces modes d'emballage.

Malgré ces précautions, les poudres commerciales de maltitol tendent à s'agglomérer en gros amas, et sont donc sensibles au mottage. Cette tendance au mottage sera d'autant plus importante que la poudre de maltitol sera de fine granulométrie.

Les poudres de maltitol cristallin ainsi mottées lors du stockage soulèvent bon nombre de problèmes, non seulement par les sérieuses difficultés de manipulation qui en découlent (transfert et déballage, broyage et remise en solution...), mais aussi en impactant fortement le rendement de ces opérations.

Un certain nombre de solutions ont été proposées pour remédier à ces difficultés :
- ajouter à la poudre de maltitol des agents antimottants tels que le stéarate de magnésium ou le talc,
- soit à glisser entre le matériau d'emballage externe et le sac interne ou à l'intérieur de ce dernier de petits sachets contenant un dessicant du type gel de silice, soit à opter pour un matériau d'emballage externe à double enveloppe, entre les enveloppes intérieure et extérieure duquel aura été préalablement insérée une substance hygroscopique.

Le premier procédé n'est cependant pas retenu par les spécialistes du domaine de la manipulation des poudres de maltitol, car il est considéré que :
- l'introduction d'impuretés dans la poudre de maltitol peut nuire à sa valeur commerciale,
- les qualités organoleptiques risquent d'être altérées et
- les contraintes réglementaires interdisent l'utilisation de tels additifs dans certaines applications.

Quant à la possible mise en oeuvre du procédé de conditionnement avec un dessicant, le risque d'une possible contamination du produit emballé par le dessicant ne peut être écarté.

Pour remédier à cette situation, dans sa demande de brevet EP 1.787.993, la société TOWA propose de traiter le maltitol cristallisé en aval des techniques de fabrication classiquement connues de l'état de la technique.

La société TOWA recommande ainsi de mettre en oeuvre divers moyens tenant compte :
- de l'équilibre entre l'humidité régnant à l'intérieur des particules et celle présente à leur surface,
- de l'équilibre entre l'humidité des particules de poudre (tant intérieure qu'en surface) et l'humidité ambiante, mais aussi
- de la stabilisation de l'état de surface de la poudre.

Dans la demande de brevet EP 1.787.993, le procédé consiste en un traitement par contact consistant à introduire dans un séchoir la poudre de maltitol cristallisé et de l'air porté à une température de 20°C à 50°C et présentant une humidité relative de 5 à 50 %, pendant cinq à cinquante heures, à une vitesse spatiale de 2 à 15 h⁻¹.

Cependant, on constate que ce procédé s'applique à des poudres de maltitol caractérisées par leur richesse très élevée en maltitol (i.e. de plus de 99,5 % tel le LESYS qui présente une teneur en maltitol de 99,7 % en poids).

Par ailleurs, si les poudres de maltitol décrites dans cette demande de brevet sont dites présenter une granulométrie inférieure à 500 µm (qualifiée de proportion de poudre passant au tamis JIS à 0,50 *mm* d'ouverture), les poudres de maltitol cristallin standard testées de type LESYS, commercialisées par la société TOWA, ou de type MALTISORB® commercialisées par ailleurs par la société Demanderesse, présentent en fait un diamètre moyen supérieur à 200 µm, et peuvent être ainsi qualifiées de poudre de maltitol cristallisé de grosse granulométrie.

Or il est bien connu de l'homme du métier que le comportement des poudres de polyols en général, et du maltitol en particulier, notamment au niveau de leur aptitude à l'écoulement, change dramatiquement en fonction de leur granulométrie : une poudre présentant un diamètre moyen de 400 à 500 µm n'aura pas le même comportement qu'une poudre de maltitol présentant un diamètre moyen de 50 à 200 µm.

Il en résulte que la solution préconisée par la société TOWA ne s'applique en fait qu'à des poudres de maltitol cristallisé de haute pureté et présentant une grosse granulométrie.

EP 1.738.657 décrit également des compositions pulvérulentes comprenant du stéarate de magnésium ou des ester d'acide gras de sucrose.

De tout ce qui précède, il résulte qu'il demeure un besoin non satisfait de disposer d'une composition de maltitol cristallisé de fine granulométrie, par exemple comprise entre 10 et 150 µm ; et présentant une richesse en maltitol variable, par exemple de 80 à 99,9 % en poids, dont la fluidité est préservée et qui présente ainsi la particularité de ne pas être sujette au mottage.

Il est du mérite de la société Demanderesse de proposer une solution technique allant à l'encontre du préjugé technique selon lequel il est fortement déconseillé d'utiliser un agent antimottant dans la préparation de composition pulvérulente de maltitol cristallisé.

La société Demanderesse recommande d'utiliser un agent antimottant. Cet agent antimottant doit être cependant choisi dans une classe particulière d'agents antimottants, plus particulièrement caractérisés par :
- leur insolubilité dans l'eau,
- leur aptitude à absorber de l'eau, et
- de préférence, leur fine granulométrie.

L'invention a trait par conséquent, en premier lieu, à une composition pulvérulente de maltitol cristallisé, caractérisée en ce :
- qu'elle présente un diamètre moyen volumique laser compris entre 10 et 150 µm ;
- qu'elle présente une richesse en maltitol comprise entre 80 et 99,9 % en poids ;
- qu'au moins 50 % en poids de ses particules s'écoulent au travers d'un tamis de seuil de coupure de 2000 µm selon un test Al ; et
- qu'au moins 35 % en poids de ses particules s'écoulent au travers d'un tamis de seuil de coupure de 2000 µm selon un test A2.

Les valeurs de répartition granulométrique sont déterminées sur un granulomètre à diffraction LASER type LS 13-320 de la société BECKMAN-COULTER, équipé de son module de dispersion poudre (voie sèche), en suivant le manuel technique et les spécifications du constructeur.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

La gamme de mesure du granulomètre à diffraction LASER type LS 13-320 est de 0,04 µm à 2.000 µm. Les résultats sont calculés en % volumique, et exprimés en µm.

La courbe de distribution granulométrique permet également de déterminer la valeur du diamètre moyen volumique (moyenne arithmétique) D4,3 (diamètre moyen volumique laser).

La composition pulvérulente de maltitol cristallisé conforme à l'invention présente ainsi un diamètre moyen volumique laser D4,3 compris entre 10 et 150 µm, de préférence 20 à 120 µm.

Selon un mode de réalisation, la composition pulvérulente de maltitol cristallisé selon l'invention présente un diamètre moyen volumique laser compris entre 50 et 90 µm ; de préférence entre 60 et 80 µm.

Selon un autre mode de réalisation, la composition pulvérulente de maltitol cristallisé selon l'invention présente un diamètre moyen volumique laser compris entre 15 et 50 µm ; de préférence entre 20 et 40 µm.

La composition pulvérulente de maltitol cristallisé selon l'invention présente une richesse en maltitol comprise entre 80 et 99,9 % en poids ; de préférence comprise entre 85 et 99.9 % en poids ; de préférence comprise entre 90 et 99.9 % en poids ; de préférence comprise entre 95 et 99.9 % en poids.

Le caractère antimottant de la composition pulvérulente de maltitol cristallisé conforme à l'invention est déterminé selon un test A de vieillissement accéléré.

Ce test met en oeuvre des sachets de polyéthylène basse densité de dimensions intérieures 10 cm x 5 cm, qui sont obtenus par thermo-soudage sur trois côtés de films de polyéthylène basse densité d'épaisseur 100 µm.

Dans ces sachets, il est introduit 100 g de la poudre à tester, puis ces sachets sont thermo-soudés hermétiquement.

Dans une première variante, dite « sans pression » (test A1) les sachets sont posés à plat, séparément, dans une enceinte climatique.

Dans une seconde variante, dite « avec pression » (test A2), il est posé sur les sachets un poids de 2 kg, ce qui correspond à une pression de 400kg/m2.

Les sachets subissent pendant 7 jours une succession de cycles de :
- 3,5 heures à 23°C sous 83% d'humidité relative (ou HR),
- 0,5 h de transition,
- 3,5 heures à 40°C sous 92% HR,
- 0,5 h de transition.

Au bout de ces 7 jours, les sachets sont ouverts et versés sur un tamis de maille de 2000 µm mis en vibration pendant 10 secondes sur son support de marque FRITSCH™ Pulverisette Type 00.502.

L'amplitude de la vibration est réglée sur 5 et est permanente.

Le résultat est exprimé en pourcentage en poids de particules qui traversent le tamis de 2000 µm.

Selon le test A1 « sans pression », au moins 50 % en poids des particules de la composition selon l'invention traversent le tamis de 2000 µm. Selon un mode de réalisation préféré, selon le test A1, au moins 60 %, de préférence au moins 65 %, de préférence au moins 70 %, de préférence au moins 75 %, de préférence au moins 80 %, de préférence au moins 85 %, de préférence au moins 90 %, de préférence au moins 95 % des particules de la composition pulvérulente de maltitol cristallisé selon l'invention traversent le tamis de 2000 µm.

Selon le test A2 « avec pression », au moins 35 % en poids des particules de la composition selon l'invention traversent le tamis de 2000 µm. Selon un mode de réalisation préféré, selon le test A2, au moins 40 %, de préférence au moins 50 %, de préférence au moins 60 %, de préférence au moins 70 %, de préférence au moins 80 %, de préférence au moins 90 %, de préférence au moins 95 % en poids des particules de la composition pulvérulente de maltitol cristallisé selon l'invention traversent le tamis de 2000 µm.

A titre comparatif, comme il sera exemplifié ci-après, une poudre de maltitol telle celle commercialisée par la société Demanderesse sous le nom de marque MALTISORB® P90 présente les résultats suivants :
- selon le test A1, seules 5,2 % des particules de maltitol cristallisé traversent le tamis de 2000 µm, et
- selon le test A2, seules 7,4 % des particules de maltitol cristallisé traversent le tamis de 2000 µm.

La composition pulvérulente de maltitol cristallisé conforme à l'invention est également caractérisée par son contenu en agent antimottant.

Ces agents antimottants sont choisis parmi les antimottants caractérisés par :
- leur insolubilité dans l'eau,
- leur aptitude à absorber de l'eau, et
- de préférence, Leur fine granulométrie.

Plus particulièrement, la composition pulvérulente de maltitol cristallisé conforme à l'invention est caractérisée en ce qu'elle comprend de 0,3 à 20 % en poids d'au moins un agent antimottant insoluble dans l'eau choisi dans le groupe .constitué par la silice pyrogénée, le silicate aluminosodique et le tri-calcium phosphate anhydre, ou de 0,5 à 20% d'un agent antimottant choisi parmi la fécule de pomme de terre déshydratée à moins de 12 % d'eau résiduelle, de préférence à moins de 10 % d'eau résiduelle, de préférence à moins de 8 % d'eau résiduelle, de préférence à 6 % d'eau résiduelle ; ledit antimottant insoluble dans l'eau présentant :
- une hygroscopicité déterminée à 80 % d'humidité relative selon un test B comprise entre 2,5 et 25 %, et
- de préférence, un diamètre moyen volumique laser inférieur à 100 µm.

Le caractère insoluble dans l'eau de l'agent antimottant est déterminé par sa structure chimique. L'homme du métier est familier avec le caractère soluble ou insoluble d'un composé dans l'eau.

L'aptitude des agents antimottants à adsorber de l'eau est déterminée selon un test B de mesure de la différence de poids exprimée en %, entre 80 % d'humidité relative et 20 % d'humidité relative à 20°C.

Ce test consiste à évaluer la variation relative de poids de l'agent antimottant lorsqu'il est soumis à un cycle d'humidité relative (dite HR) à 20°C dans un équipement fabriqué par la société Surface Measurement Systems^{™} (Londres UK) et dénommé Dynamic Vapour Sorption Série 1.

Cet équipement comprend une microbalance différentielle qui permet de quantifier l'évolution pondérale d'un échantillon par rapport à une référence (ici la nacelle de référence de la balance différentielle est vide) lorsque celui-ci est soumis à différentes conditions climatiques.

Le gaz vecteur est l'azote, et le poids de l'échantillon est inférieur à 10 mg.

A la température constante de 20°C, chaque agent antimottant subit le cycle d'humidité relative suivant : 5 heures à 20 %, montée de 20 % à 70 % en 30 minutes, maintien 5 heures à 70 %, montée de 70 % à 80 % en 30 minutes, et maintien 5 heures à 80 %.

L'hygroscopicité à 80% H.R. est alors donnée par la relation : (m₈₀-m₂₀)/m₂₀×100 exprimée en %, où m₂₀ est la masse de l'échantillon à la fin des 5 heures à 20% H.R. et m₈₀, la masse de l'échantillon à la fin des 5 heures à 80% H.R.

Les agents antimottants qui sont mis en oeuvre dans la composition pulvérulente de maltitol cristallisé conforme à l'invention présentent une hygroscopicité déterminée à 80 % d'humidité relative selon un test B comprise entre 2,5 et 25 %

A titre de comparaison, d'autres agents antimottants classiquement choisis, tel le stéarate de magnésium, ou le talc, présentent une très faible aptitude à adsorber de l'eau, leurs valeurs d'hygroscopicité à 80 % mesurée selon le test B sont en effet comprises entre 0,05 et 1,0 %.

Les agents antimottants de la composition pulvérulente de maltitol cristallisé conforme à l'invention sont également caractérisés par leur granulométrie, mesurée sur un granulomètre à diffraction LASER type LS 13-320 de la société BECKMAN-COULTER, équipé de son module de dispersion poudre (voie sèche), en suivant le manuel technique et les spécifications du constructeur, comme indiqué ci-avant.

Selon l'invention, les agents antimottants présentent un diamètre moyen volumique laser inférieur à 100 µm. Préférentiellement, selon l'invention, l'agent antimottant présente un diamètre moyen volumique laser inférieur à 80 µm, plus préférentiellement inférieur à 50 µm, plus préférentiellement inférieur à 40 µm, plus préférentiellement inférieur à 30 µm, plus préférentiellement inférieur à 10 µm.

Selon un mode de réalisation, la composition pulvérulente de maltitol cristallisé selon l'invention comprend de 0,3 à 15 %, de préférence de 0,3 à 10 %, de préférence de 0,3 à 5 % en poids d'un agent antimottant. choisi dans le groupe constitué par la silice pyrogénée, le silicate aluminosodique, et le tri-calcium phosphate anhydre; de 0,5 à 15 %, de préférence de 0,5 à 10 %, de préférence de 0,5 à 5 % en poids d'un agent antimottant choisi parmi la fécule de pomme de terre déshydratée à moins de 12 % d'eau résiduelle, de préférence à moins de 10 % d'eau résiduelle, de préférence à moins de 8 % d'eau résiduelle, de préférence à 6 % d'eau résiduelle.

Selon un premier mode de réalisation de ce mode préférentiel, la composition pulvérulente de maltitol cristallisé conforme à l'invention comprend de 0,3 à 3 % en poids, de préférence de l'ordre de 0,5 à 2 % en poids de silice pyrogénée ou de silicate aluminosodique.

Selon un deuxième mode de réalisation de ce mode préférentiel, la composition pulvérulente de maltitol cristallisé conforme à l'invention comprend de 0,3 à 3 % en poids, de préférence de l'ordre de 2 % en poids de tri-calcium phosphate anhydre.

Selon un troisième mode de réalisation de ce mode préférentiel, la composition pulvérulente de maltitol cristallisé conforme à l'invention comprend de 0.5 à 20 % en poids, de préférence de l'ordre de 5 % en poids de fécule déshydratée à moins de 12 % d'eau résiduelle, de préférence à moins de 10 % d'eau résiduelle, de préférence à moins de 8 % d'eau résiduelle, de préférence à 6 % d'eau résiduelle.

Comme il sera exemplifié ci-après, ces agents antimottants respectent les propriétés pré-requises relatives à :
- leur insolubilité dans l'eau,
- leur aptitude à absorber de l'eau, et
- de préférence, leur fine granulométrie.

La présente invention concerne également l'utilisation d'au moins un tel agent antimottant insolubles dans l'eau, présentant :
- une hygroscopicité déterminée selon le test B comprise entre 2,5 et 25 %, et
- de préférence, un diamètre moyen volumique laser inférieur à 100 µm,
pour la préparation d'une composition pulvérulente de maltitol cristallisé.

Selon un mode de réalisation, la présente invention concerne également l'utilisation d'au moins un agent antimottant choisi parmi :
- la silice pyrogénée,
- le silicate aluminosodique,
- le tri-calcium phosphate anhydre et,
- la fécule de pomme de terre déshydratée (notamment fécule de pomme de terre déshydratée à moins de 12 % d'eau résiduelle, de préférence à moins de 10 % d'eau résiduelle, de préférence à moins de 8 % d'eau résiduelle, de préférence à 6 % d'eau résiduelle),
pour la préparation d'une composition pulvérulente de maltitol cristallisé.

Selon un mode de réalisation, la composition pulvérulente de maltitol cristallisé selon l'invention est susceptible d'être obtenue par mélange :
- de maltitol cristallisé présentant un diamètre moyen volumique laser compris entre 10 et 150 µm ; et
- d'au moins un agent antimottant insoluble dans l'eau, ledit agent antimottant présentant une hygroscopicité déterminée selon le test B comprise entre 2,5 et 25 %, et présentant de préférence un diamètre moyen volumique laser inférieur à 100 µm.

Selon un mode de réalisation, dans la composition pulvérulente de maltitol cristallisé conforme à l'invention, le diamètre moyen volumique laser de l'agent antimottant est inférieur au diamètre moyen volumique laser du maltitol cristallisé.

Selon un mode de réalisation préféré, la composition pulvérulente de maltitol cristallisé selon l'invention est susceptible d'être obtenue par mélange :
- de maltitol cristallisé présentant un diamètre moyen volumique laser compris entre 50 et 90 µm ; de préférence entre 60 et 80 µm ; par exemple du MALTISORB™ P90 commercialisé par la société Demanderesse ; et
- d'au moins un agent antimottant comme indiqué ci-dessus.

Selon un autre mode de réalisation préféré, la composition pulvérulente de maltitol cristallisé selon l'invention est susceptible d'être obtenue par mélange :
- de maltitol cristallisé présentant un diamètre moyen volumique laser compris entre 15 et 50 µm ; de préférence entre 20 et 40 µm ; par exemple du MALTISORB™ P35 commercialisé par la société Demanderesse ; et
- d'au moins un agent antimottant comme indiqué ci-dessus.

La composition pulvérulente de maltitol cristallisé conforme à l'invention est susceptible d'être obtenue en mettant en oeuvre toute méthode connue par ailleurs de l'homme du métier, comprenant une étape de mélange physique des deux poudres.

L'invention concerne également l'utilisation d'une composition pulvérulente de mannitol cristallisé telle que décrite ci-dessus pour le remplissage de conteneurs souples, par exemple des « big bags ».

Par ailleurs, la composition pulvérulente selon l'invention est utile dans les domaines alimentaires et/ou pharmaceutiques.

Ainsi, la présente invention concerne également l'utilisation d'une composition pulvérulente de mannitol cristallisé telle que décrite ci-dessus, pour la préparation de compositions alimentaires, notamment des confiseries telles que des gommes à mâcher (chewing gums), des chocolats, des édulcorants de table, des biscuits, des crèmes glacées, des boissons en poudre, des entremets, des préparations pour gâteaux ou des poudres chocolatées ou vanillées pour petit déjeuner ; et/ou pour la préparation de compositions pharmaceutiques, notamment de formes sèches pharmaceutiques telles que des gélules, des médicaments du type poudres à dissoudre, des comprimés et des préparations nutritives pulvérulentes à diluer

L'invention sera encore mieux comprise à l'aide des exemples qui suivent, lesquels ne se veulent pas limitatifs et font seulement état de certains modes de réalisation et de certaines propriétés avantageuses de la poudre de maltitol cristallisé selon l'invention.

### Exemples

### Exemple 1

On prépare quatre compositions pulvérulentes de maltitol cristallisé par mélange physique d'une poudre de maltitol cristallisé commercialisée par la société Demanderesse sous le nom de marque MALTISORB® P90, présentant un diamètre moyen volumique D4,3 de 69.5 µm avec 4 agents antimottants :
- la silice pyrogénée AEROSIL™ 200, commercialisée par la société EVONIK - DEGUSSA GmbH.
- le silicate aluminosodique DURAFILL™ 200, commercialisée par la société W.R. GRACE & CO,
- le tri-calcium phosphate anhydre ou TCP, commercialisé par la société PRAYON S.A.,
- la fécule de pomme de terre déshydratée 6% d'eau résiduelle, commercialisée par la société Demanderesse sous ce même nom.

Ces 4 agents antimottants présentent les caractéristiques d'adsorption d'eau et de granulométrie figurées dans le tableau 1 suivant :

**Tableau 1**

| | Hygroscopicité à 80 % H.R. selon le test B (%) | Diamètre moyen volumique laser D4,3 (µm) |
|---|---|---|
| Silice pyrogénée AEROSIL 200 | 3,09 | 0,012 |
| Silicate aluminosodique DUPAFILL 200 | 5,48 | 4,91 |
| TCP | 3,47 | 7,16 |
| Fécule de pomme de terre déshydratée | 15,06 | 35,96 |

Les teneurs en poudre de maltitol et en agent antimottant de ces 4 compositions pulvérulentes de maltitol cristallisé conformes à l'invention sont présentées dans le tableau 2 suivant :

**Tableau 2**

| | Composition « A » | Composition « B » | Composition « C » | Composition « D » |
|---|---|---|---|---|
| MALTISORB® P90 (% en poids) | 98 | 98 | 98 | 95 |
| Silice pyrogénée AEROSIL™ 200 (% en poids) | 2 | | | |
| Silicate aluminosodique DURAFILL™ 200 (% en poids) | | 2 | | |
| TCP (% en poids) | | | 2 | |
| Fécule de pomme de terre déshydratée (% en poids) | | | | 5 |

Les compositions « A », « B », « C » et « D » selon l'invention présentent un diamètre moyen volumique laser compris entre 10 et 150 µm.

Le caractère antimottant de ces 4 compositions pulvérulentes de maltitol cristallisé est déterminé à l'aide du test A de vieillissement accéléré.

Les valeurs obtenues sont présentées dans le tableau 3 suivant :

**Tableau 3**

| | Composition « A » | Composition « B » | Composition « C » | Composition « D » |
|---|---|---|---|---|
| Test A1 Test de vieillissement accéléré sans pression (%) | 83,9 | 68,7 | 82,3 | 97,6 |
| Test A2 Test de vieillissement accéléré avec pression (%) | 72,3 | 48,9 | 35,4 | 98,6 |

Ces résultats sont comparés avec des mesures effectuées avec des agents antimottants de type stéarate de magnésium (Mg stearate Ph./Veg de Wiga Pharma Gmbh) et talc (Talc extra sup DEC de Talc Luzenac) dont les paramètres d'adsorption d'eau et de granulométrie sont donnés dans le tableau 4 suivant.

**Tableau 4**

| | Hygroscopicité à 80 % H.R. selon le test B (%) | Diamètre moyen volumique laser D4,3 (µm) |
|---|---|---|
| Stéarate de Magnésium | 0,86 | 11,1 |
| Talc | 0,08 | 19,9 |

Les mesures effectuées selon le test A de vieillissement accéléré des deux compositions témoins « E » (99 % de MALTISORB® P90 et 1% de Stéarate de Magnésium) et « F » (98 % de MALTISORB® P90 et 2 % de Talc) sont présentées dans le tableau 5 suivant.

**Tableau 5**

| | Composition « E » | Composition F » |
|---|---|---|
| Test A1 Test de vieillissement accéléré sans pression | 8,5% | 3,5% |
| Test A2 Test de vieillissement accéléré avec pression | 6,9% | 8,2% |

Les compositions pulvérulentes de maltitol cristallisé conformes à l'invention présentent donc des propriétés remarquables d'écoulement.

### Exemple 2

On prépare une composition pulvérulente de maltitol cristallisé par mélange physique d'une poudre de maltitol cristallisé commercialisé par la société Demanderesse sous le nom de marque MALTISORB® P90, présentant un diamètre moyen volumique D4,3 de 77,8 µm avec un agent antimottant, la fécule de pomme de terre déshydratée qui présente un teneur en eau de 6.0%. En final, ce mélange pulvérulent comprend 95% de poudre de maltitol et 5% de fécule déshydratée. La composition présente un diamètre moyen volumique laser compris entre 10 et 150 µm.

A partir du même lot de MALTISORB® P90, on prépare un mélange physique avec du stéarate de magnésium, de manière à obtenir un mélange pulvérulent comprenant 98% de poudre de maltitol et 2% de stéarate de magnésium. La composition présente un diamètre moyen volumique laser compris entre 10 et 150 µm.

Avec chacun de ces deux mélanges physiques et le lot de MALTISORB® P90 initial, on remplit trois conteneurs souples type 66S contenant une sache en polyéthylène d'épaisseur 100 µm, avec 800kg de poudre.

Ces trois conteneurs sont fermés simultanément et de manière identique. Ensuite ces trois conteneurs souples sont placés côte à côte, sur des palettes en bois, et sont conservés pendant un an dans un même entrepôt non chauffé et non isolé. Ils subissent donc les variations de température et d'humidité liées au changement naturel du climat, mais aussi les variations habituelles entre les périodes de jour et de nuit.

Après un an de stockage, les conteneurs souples sont repris sur une installation de vidange adaptée à ce type de contenant et on observe la facilité à laquelle ils se vident :
- pour la poudre de MALTISORB® P90 seule : après ouverture de la goulotte de vidange, aucune poudre ne s'écoule. Le produit est complètement pris en masse et il sera impossible de vider ce container souple.
- pour le mélange avec le stéarate de magnésium : après ouverture de la goulotte de vidange, le produit s'écoule très mal et rapidement de nombreuses et grosses mottes viennent obstruer la goulotte de vidange. Il faudra de nombreuses interventions manuelles pour vider complètement le conteneur avec une perte conséquente de produit. Le produit récupéré ne pourra pas être utilisé tel quel dans son application finale parce qu'il présente trop de mottes dures et que sa granulométrie est très différente de sa granulométrie initiale.
- pour le mélange avec fécule déshydratée : après ouverture de la goulotte de vidange, la poudre s'écoule très rapidement et régulièrement. La poudre récupérée présente quelques petites mottes très friables et sa granulométrie est peu différente de celle d'origine. Le mélange pourra être utilisé tel quel et sans souci dans son application finale.

## Revendications

1. Composition pulvérulente de maltitol cristallisé, **caractérisée en ce :**
- **qu'**elle présente un diamètre moyen volumique laser compris entre 10 et 150 µm ;
- **qu'**elle présente une richesse en maltitol comprise entre 80 et 99,9 % en poids ;
- **qu'**au moins 50 % en poids de ses particules s'écoulent au travers d'un tamis de seuil de coupure de 2000 µm selon un test A1 ; ledit test A1 consistant à :
- introduire 100 grammes de ladite composition dans des sachets de polyéthylène basse densité de dimensions intérieures 10 cm x 5 cm obtenus par thermo-soudage sur trois côtés de films de polyéthylène basse densité d'épaisseur 100 um, lesdits sachets étant thermo-soudés hermétiquement
- poser les sachets, séparément, dans une enceinte climatique
- les sachets subissant pendant 7 jours une succession de cycles de :
- 3,5 heures à 23°C sous 83% d'humidité relative (ou HR),
- 0,5 heure de transition,
- 3,5 heures à 40°C sous 92% HR,
- 0,5 heure de transition
- les sachets étant ensuite ouverts et versés sur un tamis de maille 2000 µm mis en vibration pendant 10 secondes sur son support de marque Fritsch Pulverisette Type 00.502, l'amplitude de la vibration étant réglée sur 5 et étant permanente
- le résultat étant alors exprimé en pourcentage en poids de particules qui traversent le tamis de 2000 µm
- **qu'**au moins 35 % en poids de ses particules s'écoulent au travers d'un tamis de seuil de coupure de 2000 µm selon un test A2 ; ledit test A2 consistant à :
- introduire 100 grammes de ladite composition dans des sachets de polyéthylène basse densité de dimensions intérieures 10 cm x 5 cm obtenus par thermo-soudage sur trois côtés de films de polyéthylène basse densité d'épaisseur 100 µm, lesdits sachets étant thermo-soudés hermétiquement
- poser les sachets, séparément, dans une enceinte climatique
- poser sur les sachets un poids de 2 kg
- les sachets subissant pendant 7 jours une succession de cycles de :
- 3,5 heures à 23°C sous 83% d'humidité relative (ou HR),
- 0,5 heure de transition,
- 3,5 heures à 40°C sous 92% HR,
- 0,5 heure de transition
- les sachets étant ensuite ouverts et versés sur un tamis de maille 2000 µm mis en vibration pendant 10 secondes sur son support de marque Fritsch Pulverisette Type 00.502, l'amplitude de la vibration étant réglée sur 5 et étant permanente
- le résultat étant alors exprimé en pourcentage en poids de particules qui traversent le tamis de 2000 µm ; et
- **qu'**elle comprend de 0,3 à 20 % en poids d'au moins un agent antimottant insoluble dans l'eau choisi dans le groupe constitué par la silice pyrogénée, le silicate aluminosodique et le tri-calcium phosphate anhydre, ou de 0,5 à 20% d'un agent antimottant choisi parmi la fécule de pomme de terre déshydratée à moins de 12 % d'eau résiduelle, de préférence à moins de 10 % d'eau résiduelle, de préférence à moins de 8 % d'eau résiduelle, de préférence à 6 % d'eau résiduelle ;
ledit agent antimottant présentant un diamètre moyen volumique laser inférieur à 100 µm et une hygroscopicité déterminée selon le test B comprise entre 2,5 et 25 % ledit test B consistant à :
- faire subir, dans l'équipement Dynamic Vapour Sorption Série 1 fabriqué par la société Surface Measurement System™, équipé d'une microbalance différentielle et où le gaz vecteur est l'azote, à moins de 10 mg de la composition, le test d'humidité relative suivant :
- 5 heures à 20°C
- Montée de 20 % à 70 % en 30 minutes
- Maintien 5 heures à 70 %
- Montée de 70 % à 80 % en 30 minutes
- Et maintien 5 heures à 80 %
- L'hygroscopicité étant alors donnée par la relation (m₈₀ - m₂₀) / m₂₀x100 exprimée en %, où m₂₀ est la masse de l'échantillon à la fin des 5 heures à 20 % HR et m₈₀ la masse de l'échantillon à la fin des 5 heures à 80 % HR.

2. Composition selon la revendication 1, **caractérisée en ce qu'**elle présente un- diamètre moyen volumique laser compris entre 50 et 90 µm ; de préférence entre 60 et 80 µm.

3. Composition selon l'une quelconque des revendications 1 ou 2, **caractérisée en ce qu'**elle présente un diamètre moyen volumique laser compris entre 15 et 50 µm ; de préférence entre 20 et 40 µm.

4. Composition selon l'une quelconque des revendications 1-2, **caractérisée en ce qu'**elle est susceptible d'être obtenue par mélange :
- de maltitol cristallisé présentant un diamètre moyen volumique laser compris entre 10 et 150 µm ; et
- d'au moins un agent antimottant insoluble dans l'eau, ledit agent antimottant présentant une hygroscopicité déterminée selon le test B comprise entre 2,5 et 25 %, et présentant de préférence un diamètre moyen volumique laser inférieur à 100 µm.

5. Composition selon la revendication 4, **caractérisée en ce que** le maltitol cristallisé présente un diamètre moyen volumique laser compris entre 50 et 90 µm ; de préférence entre 60 et 80 µm.

6. Composition selon la revendication 4, **caractérisée en ce que** le maltitol cristallisé présente un diamètre moyen volumique laser compris entre 15 et 50 µm ; de préférence entre 20 et 40 µm.

7. Composition selon l'une quelconque des revendications 1-6, **caractérisée en ce qu'**elle comprend de 0,3 à 3 % en poids, de préférence environ de 0,5 à 2 % en poids de silice pyrogénée ou de silicate aluminosodique.

8. Composition selon l'une quelconque des revendications 1-6, **caractérisée en ce qu'**elle comprend de 0,3 à 3 % en poids, de préférence environ 2 % en poids de tri-calcium phosphate anhydre.

9. Composition selon l'une quelconque des revendications 1-6, **caractérisée en ce qu'**elle comprend de 0,5 à 20 % en poids, de préférence environ 5 % en poids de fécule déshydratée à 6 % d'eau résiduelle.

10. Utilisation d'au moins un agent antimottant insoluble dans l'eau choisi dans le groupe constitué par la silice pyrogénée, le silicate aluminosodique, le tri-calcium phosphate anhydre et la fécule de pomme de terre déshydratée déshydratée à moins de 12 % d'eau résiduelle, de préférence à moins de 10 % d'eau résiduelle, de préférence à moins de 8 % d'eau résiduelle, de préférence à 6 % d'eau résiduelle, et leurs mélanges et présentant :
- une hygroscopicité déterminée selon le test B comprise entre 2,5 et 25 %, et
- de préférence, un diamètre moyen volumique laser inférieur à 100 µm,
pour la préparation d'une composition pulvérulente de maltitol cristallisé selon l'une quelconque des revendications 1-9.

11. Utilisation de la composition selon l'une quelconque des revendications 1-9 pour le remplissage de conteneurs souples.

12. Utilisation d'une composition selon l'une quelconque des revendications 1-9 pour la préparation de compositions alimentaires, notamment des confiseries telles que des gommes à mâcher, des chocolats, des édulcorants de table, des biscuits, des crèmes glacées, des boissons en poudre, des entremets, des préparations pour gâteaux ou des poudres chocolatées ou vanillées pour petit déjeuner ; et/ou pour la préparation de compositions pharmaceutiques, notamment de formes sèches pharmaceutiques telles que des gélules, des médicaments du type poudres à dissoudre, des comprimés et des préparations nutritives pulvérulentes à diluer.

## Patentansprüche

1. Pulverförmige und kristalline Maltitolzusammensetzung, **dadurch gekennzeichnet, dass**:
- sie einen mittleren Laservolumendurchmesser von 10 bis 150 µm aufweist;
- sie einen Maltitolgehalt zwischen 80 und 99,9 Gewichtsprozent aufweist;
- mindestens 50 Gewichtsprozent ihrer Partikel durch ein Sieb mit einer Ausschlussgrenze von 2000 µm gemäß einem Test A1 strömen; wobei der Test A1 aus folgenden Schritten besteht:
- Einführen von 100 Gramm der Zusammensetzung in Beutel aus Polyethylen niedriger Dichte mit Innenabmessungen von 10 cm x 5 cm, die durch Heißsiegeln auf drei Seiten von Folien aus Polyethylen niedriger Dichte und mit einer Dicke von 100 µm erzielt werden, wobei die Beutel hermetisch heißversiegelt werden;
- getrenntes Legen der Beutel in eine Klimakammer;
- wobei die Beutel 7 Tage lang einer Reihe von Zyklen unterzogen werden:
- 3,5 Stunden auf 23 °C bei 83 % relativer Feuchtigkeit (bzw. HR),
- 0,5 Stunde Übergang,
- 3,5 Stunden auf 40 °C bei 92 % HR,
- 0,5 Stunde Übergang
- wobei die Beutel anschließend geöffnet werden und auf ein Sieb mit einer Maschengröße von 2000 µm ausgeschüttet werden, das 10 Sekunden lang auf seinem Träger der Marke Fritsch Pulverisette Typ 00.502 in Schwingung versetzt wird, wobei die Amplitude der Schwingung auf 5 eingestellt wird und dauerhaft ist;
- wobei das Ergebnis dann in Gewichtsprozent von Partikeln, die durch das 2000µm-Sieb gehen, ausgedrückt wird;
- mindestens 35 Gewichtsprozent ihrer Partikel durch ein Sieb mit einer Ausschlussgrenze von 2000 µm gemäß einem Test A2 strömen; wobei der Test A2 aus folgenden Schritten besteht:
- Einführen von 100 Gramm der Zusammensetzung in Beutel aus Polyethylen niedriger Dichte mit Innenabmessungen von 10 cm x 5 cm , die durch Heißsiegeln auf drei Seiten von Folien aus Polyethylen niedriger Dichte und mit einer Dicke von 100 µm erzielt werden, wobei die Beutel hermetisch heißversiegelt werden;
- getrenntes Legen der Beutel in eine Klimakammer;
- Legen eines Gewichts von 2 kg auf die Beutel;
- wobei die Beutel 7 Tage lang einer Reihe von Zyklen unterzogen werden:
- 3,5 Stunden auf 23 °C bei 83 % relative Feuchtigkeit (bzw. HR),
- 0,5 Stunde Übergang,
- 3,5 Stunden auf 40 °C bei 92 % HR,
- 0,5 Stunde Übergang
- wobei die Beutel anschließend geöffnet werden und auf ein Sieb mit einer Maschengröße von 2000 µm ausgeschüttet werden, das 10 Sekunden lang auf seinem Träger der Marke Fritsch Pulverisette Typ 00.502 in Schwingung versetzt wird, wobei die Amplitude der Schwingung auf 5 eingestellt wird und dauerhaft ist;
- wobei das Ergebnis dann in Gewichtsprozent von Partikeln, die durch das 2000um-Sieb gehen, ausgedrückt wird; und
- sie 0,3 bis 20 Gewichtsprozent mindestens eines in Wasser unlöslichen, Klumpen verhindernden Mittels, das aus der Gruppe ausgewählt wird, die aus pyrogener Kieselsäure, Aluminium-Natriumsilicat und wasserfreiem Trikalziumphosphat besteht, oder 0,5 bis 20 % eines Klumpen verhindernden Mittels, das aus dehydratisierter Kartoffelstärke mit weniger als 12 % Restwasser, bevorzugt weniger als 10 % Restwasser, bevorzugt weniger als 8 % Restwasser, bevorzugt weniger als 6 % Restwasser, ausgewählt wird, umfasst;
wobei das Klumpen verhindernde Mittel einen mittleren Laservolumendurchschnitt von weniger als 100 µm und eine Hygroskopizität aufweist, die durch den Test B bestimmt wird und zwischen 2,5 et 25 % liegt, wobei der Test B aus folgenden Schritten besteht:
- in dem Gerät Dynamic Vapour Sorption Reihe 1, das von der Firma Surface Measurement System™ hergestellt wird und mit einer Differenzialmikrowaage ausgestattet ist und bei dem das Vektorgas Stickstoff ist, Aussetzen von weniger als 10 mg der Zusammensetzung dem folgenden relativen Feuchtigkeitstest:
- 5 Stunden auf 20 °C;
- Anstieg von 20 % auf 70 % in 30 Minuten;
- 5 Stunden lang Halten auf 70 %;
- Anstieg von 70 % auf 80 % in 30 Minuten;
- und 5 Stunden lang Halten auf 80 %,
- wobei die Hygroskopizität dann durch die Beziehung (m₈₀ - m₂₀) / m₂₀x100, in % ausgedrückt, gegeben ist, wobei m₂₀ die Masse der Probe nach Ablauf der 5 Stunden bei 20 % HR und m₈₀ die Masse der Probe nach Ablauf der 5 Stunden bei 80 % HR ist.

2. Zusammensetzung nach Anspruch 1, **dadurch gekennzeichnet, dass** sie einen mittleren Laservolumendurchmesser zwischen 50 und 90 µm, bevorzugt zwischen 60 und 80 µm, aufweist.

3. Zusammensetzung nach einem der Ansprüche 1 oder 2, **dadurch gekennzeichnet, dass** sie einen mittleren Laservolumendurchmesser zwischen 15 und 50 µm, bevorzugt zwischen 20 und 40 µm, aufweist.

4. Zusammensetzung nach einem der Ansprüche 1 bis 2, **dadurch gekennzeichnet, dass** sie erzielt werden kann durch Mischen von:
- kristallisiertem Maltitol, das einen mittleren Laservolumendurchschnitt zwischen 10 und 150 µm aufweist; und
- mindestens einem in Wasser unlöslichen, Klumpen verhindernden Mittel, wobei das Klumpen verhindernde Mittel eine Hygroskopizität aufweist, die gemäß dem Test B bestimmt wird und zwischen 2,5 und 25 % liegt, und bevorzugt einen mittleren Laservolumendurchschnitt von weniger als 100 µm aufweist.

5. Zusammensetzung nach Anspruch 4, **dadurch gekennzeichnet, dass** das kristallisierte Maltitol einen mittleren Laservolumendurchmesser zwischen 50 und 90 µm, bevorzugt zwischen 60 und 80 µm, aufweist.

6. Zusammensetzung nach Anspruch 4, **dadurch gekennzeichnet, dass** das kristallisierte Maltitol einen mittleren Laservolumendurchmesser zwischen 15 und 50 µm, bevorzugt zwischen 20 und 40 µm, aufweist.

7. Zusammensetzung nach einem der Ansprüche 1 bis 6, **dadurch gekennzeichnet, dass** sie 0,3 bis 3 Gewichtsprozent, bevorzugt ungefähr 0,5 bis 2 Gewichtsprozent, von pyrogener Kieselsäure oder Aluminium-Natriumsilicat umfasst.

8. Zusammensetzung nach einem der Ansprüche 1 bis 6, **dadurch gekennzeichnet, dass** sie 0,3 bis 3 Gewichtsprozent, bevorzugt ungefähr 2 Gewichtsprozent, von wasserfreiem Trikalziumphosphat umfasst.

9. Zusammensetzung nach einem der Ansprüche 1 bis 6, **dadurch gekennzeichnet, dass** sie von 0,5 bis 20 Gewichtsprozent, bevorzugt ungefähr 5 Gewichtsprozent, von dehydratisierter Kartoffelstärke mit 6 % Restwasser umfasst.

10. Verwendung mindestens eines in Wasser unlöslichen, Klumpen verhindernden Mittels, das aus der Gruppe, die aus pyrogener Kieselsäure, Aluminium-Natriumsilicat, wasserfreiem Trikalziumphosphat und dehydratisierter Kartoffelstärke mit weniger als 12 % Restwasser, bevorzugt weniger als 10 % Restwasser, bevorzugt weniger als 8 % Restwasser, bevorzugt 6 % Restwasser, und Mischungen derselben besteht, gewählt wird und Folgendes aufweist:
- eine Hygroskopizität, die gemäß dem Test B bestimmt wird und zwischen 2,5 und 25 % liegt, und
- bevorzugt einen mittleren Laservolumendurchschnitt von weniger als 100 µm,
für die Zubereitung einer pulverförmigen Zusammensetzung aus kristallisiertem Maltitol nach einem der Ansprüche 1 bis 9.

11. Verwendung der Zusammensetzung nach einem der Ansprüche 1 bis 9 zum Befüllen von flexiblen Behältern.

12. Verwendung einer Zusammensetzung nach einem der Ansprüche 1 bis 9 für die Zubereitung von Lebensmittelzusammensetzungen, insbesondere von Süßigkeiten, wie etwa Kaugummi, Pralinen, Tafelsüßstoffen, Kekse, Speiseeis, Getränkepulvern, Süßspeisen, Kuchenmischungen oder Pulver mit Schokoladen- oder Vanillegeschmack zum Frühstück; und/oder für die Zubereitung von pharmazeutischen Zusammensetzungen, insbesondere von pharmazeutischen trockenen Formen, wie Kapseln, Medikamente nach Art von löslichen Pulvern, Tabletten und zu verdünnende pulverförmige Ernährungszubereitungen.

## Claims

1. Pulverulent crystalline maltitol composition, **characterized in that**:
- it has a laser volume mean diameter between 10 and 150 µm;
- it has a maltitol content between 80 and 99.9 wt%;
- at least 50 wt% of its particles flow through a sieve having a cut-off threshold of 2000 µm according to a test A1; said test A1 consisting in:
- introducing 100 g of said composition in low-density polyethylene sachets with internal dimensions of 10 cm x 5 cm, which are obtained by heat-sealing low-density polyethylene films having a thickness of 100 µm along three sides said sachets being hermetically heat-sealed
- laying flat the sachets, separately, in a climatic chamber
- the sachets undergoing over 7 days a succession of cycles of:
- 3.5 hours at 23°C in 83% relative humidity (or RH) ;
- 0.5 h of transition;
- 3.5 hours at 40°C in 92% RH; and
- 0.5 h of transition.
- the sachets being then opened and poured over a sieve having a mesh of 2000 µm, vibrated for 10 seconds over its trademark FRITSCH™ Pulverisette Type 00.502 support, the amplitude of the vibration being set to 5 and being stable.
- the result being expressed as the percentage by weight of particles that pass through the 2000 µm sieve
- at least 35 wt% of its particles flow through a sieve having a cut-off threshold of 2000 µm according to a test A2; said test A2 consisting in:
- introducing 100 g of said composition in low-density polyethylene sachets with internal dimensions of 10 cm x 5 cm, which are obtained by heat-sealing low-density polyethylene films having a thickness of 100 µm along three sides said sachets being hermetically heat-sealed
- laying flat the sachets, separately, in a climatic chamber
- placing a 2 kg weight on the sachets
- the sachets undergoing over 7 days a succession of cycles of:
- 3.5 hours at 23°C in 83% relative humidity (or RH) ;
- 0.5 h of transition;
- 3.5 hours at 40°C in 92% RH; and
- 0.5 h of transition.
- the sachets being then opened and poured over a sieve having a mesh of 2000 µm, vibrated for 10 seconds over its trademark FRITSCH™ Pulverisette Type 00.502 support, the amplitude of the vibration being set to 5 and being stable.
- the result being expressed as the percentage by weight of particles that pass through the 2000 µm sieve; and
- it comprises from 0.3 to 20 w% of at least one water-insoluble anti-caking agent chosen from the group composed of fumed silica, sodium aluminosilicate and anhydrous tricalcium phosphate, or from 0.5 to 20 % of an anti-cacking agent chosen from dehydrated potato starch having less than 12% residual water, preferably having less than 10% residual water, preferably having less than 8% residual water, preferably having 6% residual water,
said anti-caking agent having a laser volume mean diameter below 100 µm and a hygroscopicity, determined according to the test B, between 2.5 and 25%, said test B consisting in:
- subjecting, in the equipment Dynamic Vapour Sorption Series 1manufactured by Surface Measurement Systems™ equipped with a differential microbalance and wherein the carrier gas is nitrogen, to less than 10 mg of the composition, the following relative humidity test:
- 5 hours at 20%
- climb from 20% to 70% over 30 minutes
- hold for 5 hours at 70%
- climb from 70% to 80% over 30 minutes
- and hold for 5 hours at 80%
- the hygroscopicity being then given by the equation: (m₈₀-m₂₀)/m₂₀×100 expressed in %, where m₂₀ is the mass of the sample at the end of the 5 hours at 20% RH and m₈₀ is the mass of the sample at the end of the 5 hours at 80% RH.

2. Composition according to Claim 1, **characterized in that** it has a laser volume mean diameter between 50 and 90 µm; preferably between 60 and 80 µm.

3. Composition according to either of Claims 1 or 2, **characterized in that** it has a laser volume mean diameter between 15 and 50 µm; preferably between 20 and 40 µm.

4. Composition according to any one of Claims 1 or 2, **characterized in that** it is capable of being obtained by mixing:
- crystalline maltitol having a laser volume mean diameter between 10 and 150 µm; and
- at least one water-insoluble anti-caking agent, said anti-caking agent having a hygroscopicity, determined according to the test B, between 2.5 and 25%, and preferably having a laser volume mean diameter below 100 µm.

5. Composition according to Claim 4, **characterized in that** the crystalline maltitol has a laser volume mean diameter between 50 and 90 µm; preferably between 60 and 80 µm.

6. Composition according to Claim 4, **characterized in that** the crystalline maltitol has a laser volume mean diameter between 15 and 50 µm; preferably between 20 and 40 µm.

7. Composition according to any one of Claims 1 to 6, **characterized in that** it comprises from 0.3 to 3 wt%, preferably around 0.5 to 2 wt% of fumed silica or of sodium aluminosilicate.

8. Composition according to any one of Claims 1 to 6, **characterized in that** it comprises from 0.3 to 3 wt%, preferably around 2 wt% of anhydrous tricalcium phosphate.

9. Composition according to any one of Claims 1 to 6, **characterized in that** it comprises from 0.5 to 20 wt%, preferably around 5 wt% of dehydrated starch having 6% residual water.

10. Use of at least one water-insoluble anti-caking agent chosen from the group composed of fumed silica, sodium aluminosilicate, anhydrous tricalcium phosphate and dehydrated potato starch having less than 12% residual water, preferably having less than 10% residual water, preferably having less than 8% residual water, preferably having 6% residual water, and mixtures thereof, and having:
- a hygroscopicity, determined according to the test B, between 2.5 and 25%; and
- preferably, a laser volume mean diameter below 100 µm, for the preparation of a pulverulent crystalline maltitol composition according to any one of claims 1 to 9.

11. Use of the composition according to any one of Claims 1 to 11, for filling flexible containers.

12. Use of a composition according to any one of Claims 1 to 11, for the preparation of food compositions, especially confectionery such as chewing gums, chocolates, table-top sweeteners, biscuits, ice creams, powdered drinks, desserts, cake preparations or chocolate or vanilla powders for breakfast; and/or for the preparation of pharmaceutical compositions, especially dry pharmaceutical forms such as gelatin capsules and medicinal products of the following types: powders to be dissolved, tablets and pulverulent nutritional preparations to be diluted.
